# EUROPÄISCHE PATENTSCHRIFT

(11) **EP 3 893 985 B1**
(45) Veröffentlichungstag und Bekanntmachung des Hinweises auf die Patenterteilung: **15.04.2026**
(21) Anmeldenummer: 19817993.9
(22) Anmeldetag: 04.12.2019
(51) Int. Cl.: A61M 37/00

(54) **VERFAHREN UND VORRICHTUNG ZUR HERSTELLUNG VON MIKRONADELN**
METHOD AND DEVICE FOR PRODUCING MICRONEEDLES
PROCÉDÉ ET DISPOSITIF DE FABRICATION DE MICRO-AIGUILLES

(30) Priorität: 11.12.2018 DE 102018009594
(43) Veröffentlichungstag der Anmeldung: 20.10.2021
(73) Patentinhaber: LTS Lohmann Therapie-Systeme AG, 56626 Andernach (DE)
(72) Erfinder: KULIK, Michael, 56220 Urmitz (DE); HENNIG, Andreas, 72469 Meßstetten (DE)
(74) Vertreter: dompatent
(86) Internationale Anmeldenummer: PCT/EP2019/083717
(87) Internationale Veröffentlichungsnummer: WO 2020/120262

(56) Entgegenhaltungen:
- EP-A1- 3 248 593
- CA-A1- 2 696 956
- JP-A- 2011 206 178
- JP-A- 2017 143 963
- KR-A- 20170 135 773
- US-A1- 2010 305 516
- US-A1- 2016 136 408

## Beschreibung

Die Erfindung betrifft ein Verfahren zum Herstellen von Mikronadelarrays in einer Form, die eine Vielzahl sich von einer obenliegenden Basisfläche zu einer untenliegenden Spitzenfläche verjüngender Aufnahmen aufweist und eine Vorrichtung zum Herstellen von Mikronadelarrays mittels eines derartigen Verfahrens mit einer Form.

Aus der EP 2 664 323 A1 ist ein derartiges Verfahren, eine Vorrichtung und ein damit hergestelltes Mikronadelarray bekannt. Hierbei wird eine, ein Medikament und Lösungsmittel enthaltende Lösung in ein Gesenk eingefüllt. Um Luftblasen zu vermeiden, erfolgt dieses Befüllen mittels Überdruck der Lösung oder Unterdruck der Umgebung.

Eine weiterer relevanter Stand der Technik für die vorliegende Erfindung ist in KR 10 2017 0135773 A beschrieben.

Der vorliegenden Erfindung liegt die Aufgabe zugrunde, bei großtechnischer Herstellung eine ausreichende pharmazeutische Qualität zu gewährleisten und eine hohe Stückzahl von Mikronadelarrays zu ermöglichen.

Die Erfindung ist in den Ansprüchen 1 und 3 definiert.

Die Problemstellung wird mit den Merkmalen des Hauptanspruchs gelöst. Mindestens zwei Aufnahmen wird durch eine von der Basisfläche beabstandete Zuführöffnung hindurch eine erste Komponente zugeführt. Diese Aufnahmen werden von oberhalb der Zuführöffnung aus mit einer weiteren Komponente befüllt. Die zumindest aus der ersten Komponente und aus der weiteren Komponente gebildeten Füllungen mindestens dieser zwei Aufnahmen werden oberhalb der Basisflächen miteinander verbunden. Außerdem wird nach dem Verfestigen der ersten Komponente und der weiteren Komponente das Mikronadelarray mit den zu Nadeln erstarrten Füllungen aus der Form entnommen.

Die bei diesem Verfahren eingesetzte Form weist eine Vielzahl von sich von einer obenliegenden Basisfläche zu einer untenliegenden Spitzenfläche verjüngender Aufnahmen auf. Jede Aufnahme hat eine von der Basisfläche beabstandete Zuführöffnung.

Im Mikronadelarray sind im Nadelanschlussquerschnitt die Nadeln mit einer Trägerplatte verbunden. Außerdem besteht der an die kleinste Stirnfläche angrenzende Bereich aus der verfestigten ersten Komponente.

Die Herstellung des Mikronadelarrays erfolgt in einer Form, die eine Vielzahl von sich von oben nach unten verjüngenden Aufnahmen hat. Diese Aufnahmen haben eine eine obenliegende Basisfläche und eine untenliegende Spitzenfläche verbindende Innenmantelfläche. Jede Aufnahme hat mindestens eine Zuführöffnung, die in der Spitzenfläche angeordnet ist. Diese Zufuhröffnung kann verschließbar ausgebildet sein.

Die Form kann bei der Herstellung des Mikroarrays nacheinander in mindestens zwei Verfahrensschritten befüllt werden. Eine erste Komponente, z.B. ein Wirkstoff, eine wirkstoffhaltige Komponente, etc., wird durch die Zuführöffnungen in die Aufnahmen eingebracht. Weiter wird beispielsweise eine zweite Komponente, z.B. eine Füllmasse, in die Aufnahmen eingebracht. Hierbei kann zunächst die erste und anschließend die zweite Komponente zugeführt werden. Es ist auch denkbar, beide Komponenten gleichzeitig zuzuführen. Beispielsweise können hierbei hochviskose Komponenten, Laminate, Pulver, etc. zugeführt werden.

Das Einbringen der weiteren Komponente erfolgt von der Basisfläche aus oder von einer oberhalb der Zuführöffnung liegenden Füllöffnung aus. Diese Füllöffnung liegt dann zwischen der Basisfläche und der Zuführöffnung. Oberhalb der Basisfläche werden die Füllungen mindestens zweier Aufnahmen miteinander verbunden. Hierbei kann die zweite Komponente oder ein anderer Werkstoff eingesetzt werden. Die zumindest aus der ersten Komponente und der zweiten Komponente bestehenden Füllungen der Aufnahmen werden verfestigt oder verfestigen sich. Es ist auch denkbar, die einzelnen Komponenten einzeln nacheinander zu verfestigen. Beispielsweise werden sie hierbei durch Reduzierung des Feuchtigkeitsgehalts getrocknet. Die einzelnen Füllungen verfestigen sich hierbei zu einzelnen starren Nadeln. Anschließend kann das Mikronadelarray mit den oberhalb der Basisflächen miteinander verbundenen Nadeln aus der Form entnommen und angewendet werden.

Gegebenenfalls kann das Mikronadelarray auch aus mehr als zwei Komponenten hergestellt werden. Die z.B. wirkstofffreie zweite Komponente kann beispielsweise unabhängig vom Werkstoff der Spitzen, z.B. ein Gemisch aus Wirk- und Hilfsstoffen, kostengünstig für eine Vielzahl von Produkten eingesetzt werden.

Das Mikronadelarray besteht mindestens aus zwei Komponenten, wobei die Spitzen der kegelstumpfförmigen Nadeln z.B. aus der verfestigten ersten, beispielsweise wirkstoffhaltigen Komponente bestehen. Diese Spitzen haben eine z.B. kreisförmige Stirnfläche. Beim Einsatz beispielsweise auf der Haut eines Patienten, dringen die biegesteifen und bruchfesten Nadeln in die Haut des Patienten ein, wobei die Spitzen die Hautbereiche unterhalb der Hornhaut erreichen. Gegebenenfalls können sich die Spitzen bei der Wirkstoffabgabe auflösen. Die Spitzen und/oder die Basis der Nadeln können dabei ein oder mehrere zur therapeutischen und/oder diagnostischen Anwendung vorgesehene Substanzen enthalten.

Weitere Einzelheiten der Erfindung ergeben sich aus den Unteransprüchen und den nachfolgenden Beschreibungen schematisch dargestellter Ausführungsbeispiele.
- Figur 1:: Form;
- Figur 2:: Unteransicht von Figur 1;
- Figur 3:: Unterschale;
- Figur 4:: Isometrische Schnittdarstellung der Vorrichtung vor dem Befüllen;
- Figur 5:: Unterschale mit eingeprägten Kanälen;
- Figur 6:: Unterschale mit eingeprägten Radialkanälen;
- Figur 7:: Einbringen des Wirkstoffs;
- Figur 8:: Verschluss der Durchbrüche der Form;
- Figur 9:: Vorrichtung nach dem Trocknen der ersten Komponente;
- Figur 10:: Einbringen der zweiten Komponente;
- Figur 11:: Mikronadelarray;
- Figur 12:: Mikronadelarray bei der Anwendung auf der Haut eines Patienten;
- Figur 13:: Mikronadelarray nach der Entnahme;
- Figur 14:: Vorrichtung mit beschichteten Oberflächen;
- Figur 15:: Exemplarische Vorrichtung mit seitlichen Zuführöffnungen;
- Figur 16:: Querschnitt aus Figur 15 im Bereich der Zuführkanäle.

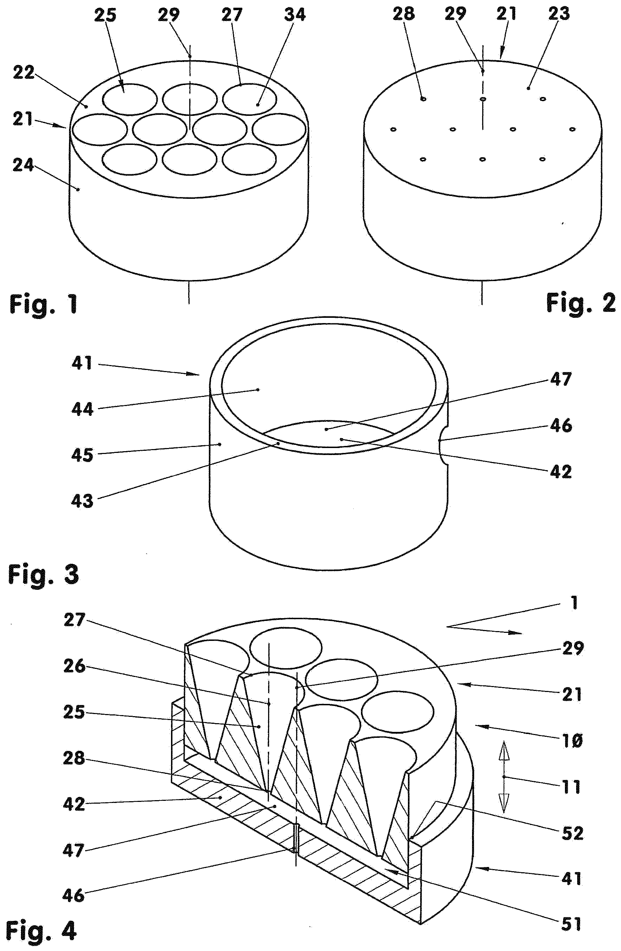

Die Figuren 1 und 2 zeigen eine Form (21), z.B. eine Gussform (21), für die Herstellung von Mikronadelarrays (61). In diesem Ausführungsbeispiel ist die Form (21) aus einem zylindrischen Körper hergestellt. Der Körper der Form (21) kann auch quaderförmig, würfelförmig, trapezoidförmigen, ellipsoidförmig, etc. ausgebildet sein. Die Form (21) kann aus einem austenitischen Stahl, einem thermoplastischen oder duroplastischen Werkstoff, etc. hergestellt sein. Sie hat in den Darstellungen der Figuren 1 und 2 eine in einer Ebene liegende kreisförmige Oberseite (22) und eine parallel zu dieser liegende ebene Unterseite (23). Eine außenliegende Mantelfläche (24) verbindet die Oberseite (22) mit der Unterseite (23). Die Form (21) hat eine Vielzahl von Aufnahmen (25). Diese Aufnahmen (25) sind in diesem Ausführungsbeispiel als Durchbrüche (25) ausgebildet, die die Oberseite (22) mit der Unterseite (23) verbinden. Im Ausführungsbeispiel sind vereinfacht 10 Durchbrüche (25) dargestellt. Die Form (21) kann z.B. pro Teilgrundfläche von 10 Quadratmillimetern beispielsweise 600 bis 700 Aufnahmen (25) aufweisen.

Die Form (21) kann auch aus mehreren, übereinanderliegenden und zueinander zentrierten Scheiben bestehen. Die einzelnen Scheiben können lösbar oder unlösbar miteinander gefügt sein. Die Aufnahmen (25) durchdringen dann z.B. mehrere oder alle Scheiben.

Die einzelne Aufnahme (25) hat im dargestellten Ausführungsbeispiel eine normal zur Oberseite (22) und zur Unterseite (23) stehende Mittellinie (26). Sie verjüngt sich kegelstumpfmantelförmig von der Oberseite (22) zur Unterseite (23). Ihre Innenmantelfläche (34) hat an jedem Punkt der Mittellinie einen z.B. kreisförmigen Querschnitt. Sowohl die in der Ebene der Oberseite (22) liegende Basisfläche (27) als auch die in der Ebene der Unterseite (23) liegende Spitzenfläche (28) der Aufnahme (25) sind z.B. kreisförmig ausgebildet. Die Mittellinie (26) verbindet die Mittelpunkte dieser beiden Kreisflächen.

Die Basisfläche (27) und/oder die Spitzenfläche (28) können eine von der Kreisform abweichende Gestalt haben. Beispielsweise können beide Flächen einen quadratischen, dreieckigen, elliptischen, etc. Querschnitt aufweisen. Beispielsweise hat dann die die einzelnen Aufnahme (25) begrenzende Wandung die Gestalt einer Pyramidenstumpfmantelfläche, eines vom Kreiskegelstumpf abweichenden Kegelstumpfabschnitts, etc. Auch ein Übergang z.B. von einem pyramidenstumpfförmigen Abschnitt in einen kegelstumpfförmigen Abschnitt ist denkbar. Die einzelne Aufnahme (25) kann auch einen an die Oberseite (22) angrenzenden Abschnitt konstanten Querschnitts aufweisen. Dieser Abschnitt kann beispielsweise zylindrisch, dreieckig, quadratisch, rechteckig, sechseckig, etc. ausgebildet sein.

Die Mittellinie (26) kann einen vom rechten Winkel abweichenden Winkel mit der Oberseite (22) und/oder der Unterseite (23) einschließen. Beispielsweise ist der Kegelstumpf dann schief ausgebildet. Es ist auch denkbar, die Basisfläche (27) und die Spitzenfläche (28) nichtparallel zueinander anzuordnen. Die Aufnahmen (25) können gleichgerichtet sein, z.B. können sie einander parallele Mittellinien (26) aufweisen.

In der Figur 3 ist eine Unterschale (41) dargestellt. Die Unterschale (41) hat einen scheibenförmigen Boden (42), der von einem ringförmigen Rand (43) begrenzt ist. Beispielsweise ist die Höhe des Randes (43) niedriger als die Höhe der Form (21) in einer Richtung normal zur Oberseite (22). Der Rand (43) hat in dieser Darstellung eine zylindermantelförmige Innenfläche (44) und eine koaxial zu dieser liegende Außenfläche (45). Der Innendurchmesser des Randes (43) ist beispielsweise um wenige Zehntel Millimeter größer als der Außendurchmesser der Form (21). Bei einer quaderförmigen Ausbildung der Form (21) ist der Rand (43) rechteckig ausgebildet. Auch in diesem Fall umgibt er die Form (21) beispielsweise mit dem genannten Abstand. Die in der Figur 3 dargestellte Unterschale (41) hat einen in der Wand (43) angeordneten Befüllanschluss (46). Der Befüllanschluss (46) kann auch im Boden (42) angeordnet sein. Er kann verschließbar ausgebildet sein. Es ist auch denkbar, die Unterschale (41) sowohl mit einem verschließbaren Befüllanschluss (46) im Rand (43) als auch mit einem verschließbaren Befüllanschluss (46) im Boden (42) auszubilden.

Die Figur 4 zeigt eine Vorrichtung (10) mit einer Form (21) und einer Unterschale (41) in einer isometrischen Schnittdarstellung. Eine Form (21) ist in die Unterschale (41) eingesetzt. Die Schnittebene verläuft durch die Mittellinien (26) von vier Aufnahmen (25) und durch die gemeinsame Mittelachse (29) der Form (21) und der Unterschale (41). Die Oberseite (22) der Form (21) mit den Basisflächen (27) der Aufnahmen (25) zeigt nach oben, die Unterseite (23) ist in Richtung des Bodens (42) der Unterschale (41) orientiert.

In dieser Darstellung der Figur 4 umgreift der Rand (43) der Unterschale (41) den unteren Bereich der Mantelfläche (24) der Form (21). Der Boden (42) der Unterschale (41) ist um einen Zuführraum (51) beabstandet zur Unterseite (23) der Form (21). Der Zuführraum (51) wird durch die Unterschale (41) und die Unterseite (23) begrenzt. Hierbei liegt der Boden (42) parallel zur Ebene der Unterseite (23). Beispielsweise ist die in der Höhenrichtung (11) orientierte Höhe des Zwischenraums (51) kleiner als fünf Millimeter. Der Ringspalt (52) zwischen der Form (21) und der Unterschale (41) kann abgedichtet sein.

Mittig im Boden (42) ist in diesem Beispiel der Befüllanschluss (46) angeordnet. Dieser durchdringt den Boden (42) und verbindet in der Darstellung der Figur 4 den Zuführraum (51) mit der Umgebung (1). Der Befüllanschluss (46) kann ein Innengewinde aufweisen. Hier kann beispielsweise ein Behälter, eine Spritze, ein Druckanschluss etc. an die Außenseite des Bodens (42) angeschraubt sein.

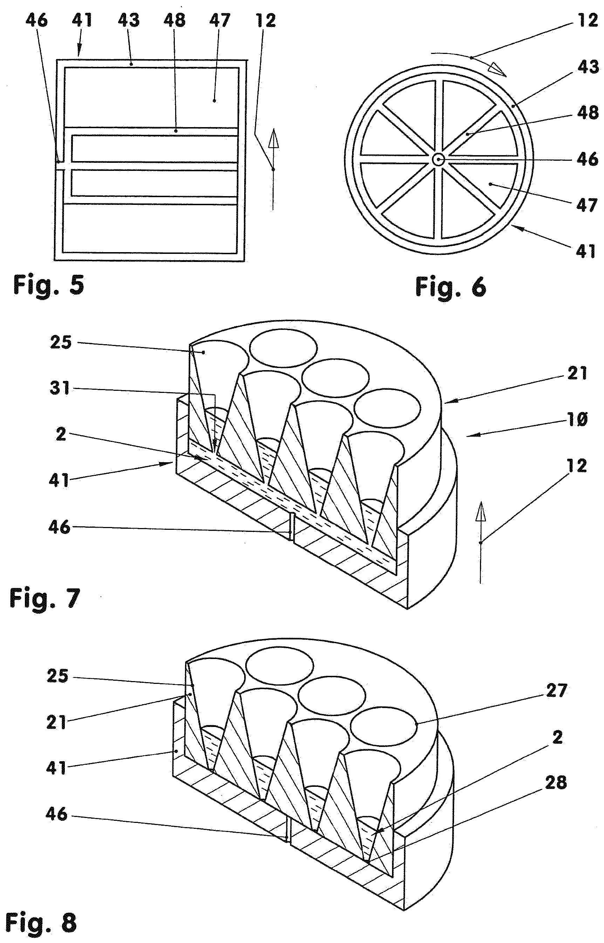

Die Form (21) und die Unterschale (41) sind relativ zueinander beweglich, z.B. verfahrbar. Im Ausführungsbeispiel sind sie zueinander in der Höhenrichtung (11) verstellbar. Der Zuführraum (51) hat damit eine variable Größe. Das Volumen des Zuführraums (51) ist in diesem Ausführungsbeispiel mittels des Hubs der Unterschale (41) relativ zur Form (21) in der Höhenrichtung (11) z.B. regelbar. In der Darstellung der Figur 4 hat der Zuführraum (51) sein maximales Betriebsvolumen. Das minimale Betriebsvolumen des Zuführraums (51) ist erreicht, wenn der Boden (42) der Unterschale (41) an der Unterseite (23) der Form (21) anliegt, vgl. Figur 8. Der Boden (42) verschließt dann die Aufnahmen (25) der Form (21) auf der Seite der Spitzenflächen (28). Die Unterschale (41) steht nun relativ zur Form (21) in einer zweiten Betriebsposition. Es ist auch denkbar, dass die Unterschale (41) eine Vielzahl, aber nicht alle Spitzenflächen (28) verschließt.

Die Vorrichtung (10) kann auch derart ausgebildet sein, dass der Boden (42) auf seiner Innenseite (47) eingeprägte Kanäle (48) aufweist, vgl. die Figuren 5 und 6. Der Boden (42) der Unterschale (41) liegt dann im Betriebszustand ständig an der Unterseite (23) der Form (21) an. Die Kanäle (48) können z.B., wie in der Figur 5 dargestellt, gerade, zueinander parallele Kanäle (48) sein, die jeweils tangential zu je einem zur in der ersten Betriebslage den Boden (42) und die Form (21) durchdringende Mittelachse (29) konzentrischen Kreis angeordnet sind. Auch eine netzförmige Ausbildung der eingeprägten Kanäle (48) ist denkbar. In diesem Fall kann die Unterschale (41) parallel zur Ebene der Unterseite (23) verfahrbar sein. Beispielsweise bei einem Verfahren in einer Verfahrrichtung (12) um eine halbe Teilung quer zur Längsrichtung der Kanäle sind die Durchbrüche (25) der Form (21) im Bereich der Spitzenflächen (28) einseitig verschließbar. Der Befüllanschluss (46) kann am Boden (42) und/oder am Rand (43) vorgesehen sein.

Die in die Innenseite (47) des Bodens (42) eingeprägten Kanäle (48) können auch radial ausgebildet sein, vgl. Figur 6. Gegebenenfalls sind zusätzliche konzentrisch um einen Mittelpunkt des Bodens (42) angeordnete Kanäle (48) vorgesehen. Die Teilung der radialen Kanäle (48) stimmt in diesem Ausführungsbeispiel mit der Teilung der Durchbrüche (25) der Form (21) überein. Im Betriebszustand liegt der Boden (42) an der Unterseite (23) der Form (21) an. Hierbei zeigt in der Ausgangsstellung jeder Durchbruch (25) der Form (21) in einen radialen Kanal außerhalb der kreisförmigen Kanäle. Der Befüllanschluss (46) kann im Mittelpunkt des Bodens (42) und/oder im Randbereich vorgesehen sein. Bei einer Anordnung im Randbereich sind die Radialkanäle beispielsweise mittels eines umlaufenden Verteilerkanals miteinander hydraulisch verbunden. Auch in diesem Ausführungsbeispiel ist die Form (21) relativ zur Unterschale (41) verfahrbar. Beispielsweise ist die Form (21) relativ zur Unterschale (41) aus der Ausgangsstellung um eine halbe kleinste Teilung um die Mittelachse (29) in einer Verfahrrichtung (12) in eine zweite Betriebsstellung verschwenkbar. Die kleinste Teilung ist diejenige Teilung, in der zwei Radialen zur Mittellinie durch zwei Durchbrüche (25) den kleinsten Winkel miteinander einschließen. In der zweiten Betriebsstellung sind dann alle Durchbrüche (25) einseitig beispielsweise mittels des Bodens (42) verschlossen.

Die Figur 7 zeigt das Einbringen einer ersten Komponente (2) in die in der Figur 4 dargestellte Vorrichtung (10). Die erste Komponente (2) ist beim Einbringen z.B. eine Flüssigkeit. Die stoffliche Zusammensetzung kann wirkstoffhaltig oder wirkstofffrei ausgebildet sein. Bei einer wirkstoffhaltigen ersten Komponente (2) kann der Wirkstoff gelöst, suspendiert oder in Form von Mikrokapseln bzw. Partikeln in die Flüssigkeit eingebettet sein. Anstatt einer flüssigen ersten Komponente (2) können auch halbfeste, pastöse oder feste Massen, Stoffmischungen oder Pulver eingebracht werden.

Die erste Komponente (2) wird beispielsweise in einem Behälter bereitgestellt, der an den bodenseitigen Befüllanschluss (46) befestigt wird. Durch Aufbringen von z.B. äußerem Druck auf den beispielsweise flexibel verformbaren Behälter wird die erste Komponente (2) in den Zuführraum (51) gefördert. Der Behälter kann auch unter Überdruck stehen. Der Zuführraum (51) wird mit der ersten Komponente (2) befüllt. Aus dem Zuführraum (51) gelangt die erste Komponente (2) durch Zufuhröffnungen (31) in die Aufnahmen (25). Die Zuführöffnungen (31) umfassen in diesem Ausführungsbeispiel die Spitzenflächen (28) der Durchbrüche (25). Beispielsweise entspricht der Flächeninhalt der einzelnen Spitzenfläche (28) der Querschnittsfläche der jeweiligen Zuführöffnung (31). Diese Querschnittsfläche ist beispielsweise kleiner oder gleich 0,01 Quadratmillimeter. Die Herstellung der Zufuhröffnungen (31) erfolgt beispielsweise mittels Laser, mittels Heißprägen, mittels Mikrofräsen, mittels Spritzgießen, mittels Lithographie, etc. In diesem Ausführungsbeispiel hat jede Aufnahme (25) genau eine Zuführöffnung (31). Alle Aufnahmen (25) werden z.B. gleichmäßig befüllt. Eventuelle Gaseinschlüsse der ersten Komponente (2) steigen als Gasblasen auf und verlassen die Aufnahmen (25) z.B. durch die Basisflächen (27). Beispielsweise werden alle Aufnahmen (25) nur bis zu einem Teilniveau befüllt. Der Füllstandsspiegel der ersten Komponente (2) in den Aufnahmen (25) liegt nach dem Befüllen in der Höhenrichtung (11) zwischen den Spitzenflächen (28) und den Basisflächen (27). Beispielsweise liegt er bei einem Drittel des Abstands der beiden genannten Flächen, gemessen von der Spitzenfläche (28) aus.

Beim Befüllen kann auch derart erfolgen, dass nur einzelne Aufnahmen (25) der Form (21) befüllt werden. Beispielsweise sind die Zuführöffnungen (31) der anderen Aufnahmen (25) z.B. temporär verschlossen. Beispielsweise nach diesem selektiven Befüllen können die restlichen Aufnahmen (25) unbefüllt verbleiben oder mit einer anderen Komponente, z.B. einer anderen wirkstoffhaltigen Lösung, befüllt werden. Hierzu können dann z.B. die zunächst befüllten Aufnahmen (25) verschlossen werden.

Das Einbringen der ersten Komponente (2) in die Aufnahmen (25) kann auch mittels Unterdruck in der Umgebung (1) der Form (21) erfolgen. Beispielsweise wird dann die z.B. fließfähige erste Komponente (2) aus dem Behälter gesaugt, der einen relativ zum Umgebungsdruck hohen Innendruck hat.

Auch können begrenzt fließfähige Massen, halbfeste bis feste Stoffmischungen oder Pulver mittels z.B. mechanischem Druck oder einer Dosierung in die Aufnahmen (25) eingebracht werden. Es ist ebenfalls denkbar, die erste Komponente (2) mittels der Wirkung von Kapillarkräften durch die Zuführöffnungen (31) in die Aufnahmen (25) zu fördern.

Im nächsten Verfahrensschritt werden die Zuführöffnungen (31) der Aufnahmen (25) verschlossen. Hierzu wird beispielsweise die Unterschale (41) relativ zur Form (21) in der Verfahrrichtung (12) verfahren, bis der Boden (42) sämtliche Durchbrüche (25) verschließt. Es ist auch denkbar, die Zuführöffnungen (31) an der Unterseite (23) der Form (21) einzeln zu verschließen, eine Verschlussplatte einzuführen, über Gelenke angeschlagene Klappen zu betätigen, etc. Auch der Einsatz einer schwenkbaren Verschlussplatte ist denkbar.

In der Figur 8 ist die in der Figur 4 dargestellte Vorrichtung mit einseitig geschlossenen Aufnahmen (25) dargestellt. Die Verbindung zwischen dem Befüllanschluss (46) der Unterschale (41) und den Aufnahmen (25) ist unterbrochen. Beispielsweise kann der Vorratsbehälter vom Befüllanschluss (46) abgenommen werden. In den nach oben offenen Aufnahmen (25) steht die erste Komponente (2). Beispielsweise hat der Füllstand in allen Aufnahmen (25) zumindest annähernd das gleiche Niveau. Die Aufnahmen (25) sind an der Oberseite (22) offen.

Anschließend wird die erste, z.B. flüssige Komponente (2) beispielsweise mittels Trocknung verfestigt. Die Unterseite (23) der Form (21) verbleibt geschlossen, die Oberseite (22) verbleibt offen. Das Trocknen kann bei gleichbleibender oder erhöhter Temperatur der Umgebung (1) erfolgen. Bei der Trocknung wird durch Verdunsten sowohl die Masse als auch das Volumen der ersten Komponente (2) in den Aufnahmen (25) vermindert. Die erste Komponente (2) verfestigt sich.

Es ist auch denkbar, die erste Komponente (2) durch eine z.B. chemische, thermische, impulsinduzierte oder strahlungsinduzierte Reaktion zu verfestigen und ggf. anschließend zu trocknen. Gegebenenfalls kann der Verfahrensschritt zur Verfestigung der ersten Komponente (2) entfallen.

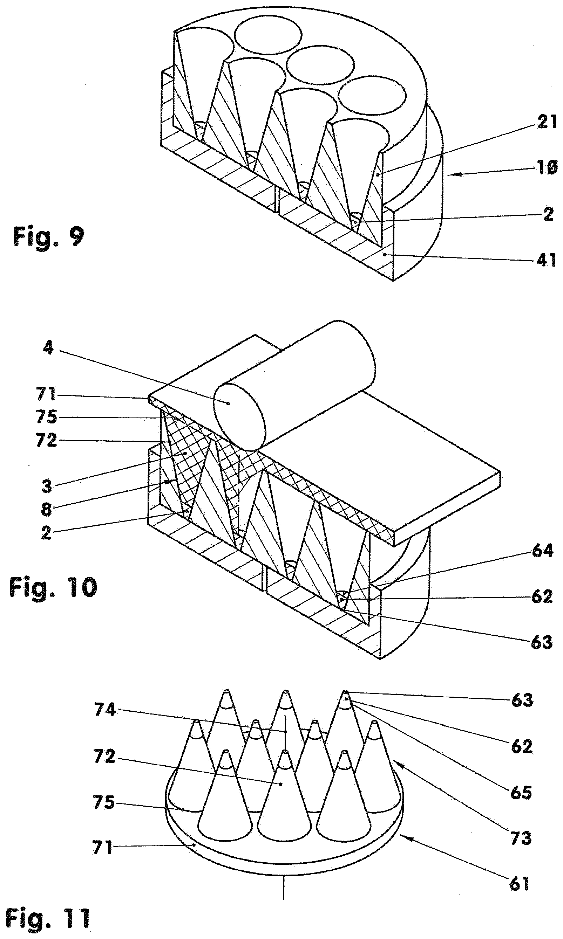

Die Figur 9 zeigt die Vorrichtung (10) mit den als Spitzen (62) verfestigten ersten Komponente (2). In jeder Aufnahme (25) sitzt, angrenzend an die z.B. mit der Unterseite (23) der Form (21) bündigen Spitzenfläche (28) eine Spitze (62). Die einzelne Spitze (62) ist kegelstumpfförmig ausgebildet. Die untenliegende, normal zur Mittellinie (26) liegende kleinste Stirnfläche (63) der Spitze (62) ist beispielsweise eine ebene Kreisfläche. Die der kleinsten Stirnfläche (62) abgewandte größte Stirnfläche (64) der Spitze (62) kann eine der kleinsten Stirnfläche (63) parallele, ebene Kreisfläche sein. Sie kann aber auch konvex oder konkav gewölbt sein. Auch kann sich durch den Trocknungsprozess bzw. den Verfestigungsprozess eine unregelmä-ßig geformte Fläche ausbilden.

Die Mantelfläche (65) der Spitzen (62) ist beispielsweise in dem an die kleinste Stirnfläche (63) angrenzenden Bereich regelmäßig ausgebildet. Beispielsweise liegt sie vollflächig an der Begrenzungsfläche der Aufnahmen (25) an. In dem an die größte Stirnfläche (64) angrenzenden Bereich der Spitze (62) kann sich die Mantelfläche (65) aufgrund der Schrumpfung beim Trocknen von der Innenwandung der einzelnen Aufnahme (25) lösen. Die Spitzen (62) sind nach dem Trocknen fest ausgebildet.

In einem weiteren Verfahrensschritt wird eine weitere, beispielsweise zweite Komponente (3), z.B. eine Füllmasse, in die Form (21) eingebracht und auf die Form (21) aufgebracht, vgl. Figur 10. Diese weitere Komponente (3) hat beispielsweise eine wirkstofffreie Formulierung. Beim Aufbringen auf die Form (21) kann die weitere Komponente (3) plastisch verformbar sein. Beispielsweise ist sie zäh verformbar ausgebildet. Beim Aufbringen wird die weitere Komponente (3) in einer dicken Schicht, z.B. bis zu einer Dicke von zehn Millimetern, auf die Oberseite (22) der Form (21) aufgetragen. Die weitere Komponente (3) ist beispielsweise unter Druck verformbar und verfestigbar. Sie kann als bahnförmiges oder plattenförmiges Laminat oder als Pulver aufgebracht werden. Auch ist der Auftrag als flüssiges Material mittels einer Pumpe denkbar. Die weitere Komponente (3) kann auch als Band oder Streifen ausgebildet sein.

Die auf die Form (21) aufgebrachte weitere Komponente (3) wird beispielsweise mittels einer Verdichtungsvorrichtung (4) in der Bauform einer Walze (4) bearbeitet. Hierbei wälzt die Walze (4) auf der Form (21) und/oder auf der weiteren Komponente (3) ab. Die weitere Komponente (3) wird in die Aufnahmen (25) gedrückt. Beim weiteren Überwalzen wird die zweite Komponente (3) sowohl in den Aufnahmen (25) als auch auf der Oberseite (22) der Form (21) verfestigt. In den Aufnahmen (25) wird die zweite Komponente (3) auf die jeweils größte Stirnfläche (64) der einzelnen Spitze (62) gepresst. Bei diesem Fügen verbindet sich beispielsweise die weitere Komponente (3) adhäsiv mit den Spitzen (62). Gegebenenfalls kann ein Klebstoffanteil in der weiteren Komponente (3) die Fügeverbindung zwischen der dieser und den Spitzen (62) verstärken. Die Füllungen (8) der Aufnahmen (25) bestehen damit jeweils zumindest aus der ersten Komponente (2) und der weiteren Komponente (3). Die weitere Komponente (3) wird z.B. durch Walzen weiter komprimiert, wobei die gesamte weitere Komponente (3) weiter verfestigt wird. Die weitere Komponente (3) besteht nun aus einem Trägerstreifen (71), z.B. einer Trägerplatte (71) und kegelstumpfförmigen Nadelstümpfen (72). Auf jedem Nadelstumpf (72) sitzt eine Spitze (62). Die weitere Komponente (3) und die Spitzen (62) sind fest und miteinander gefügt. Jeweils ein Nadelstumpf (72) ist mit einer Spitze (62) verbunden. Sie bilden zusammen mit dem Trägerstreifen (71) ein Mikronadelarray (61).

Die weitere Komponente (3) kann bereits beim Aufbringen auf die Form (21) teilweise in die Aufnahmen (25) eingebracht werden. Dies kann durch Vergießen, Spitzen, Streichen, Walzen, etc. erfolgen. Zum Verfestigen der weiteren Komponente (3) können auch Stempel eingesetzt werden. Auch ist es denkbar, die weitere Komponente (3) zum Verfestigen zu trocknen. Auch Kombinationen der genannten Verfahren sind denkbar.

Die weitere Komponente (3) kann auch unmittelbar nach dem Einbringen der ersten Komponente (2) oder zeitgleich mit dem Einbringen der ersten Komponente (2) in die Aufnahmen (25) gefördert werden. In diesem Fall erfolgt beispielsweise z.B. keine separate Verfestigung oder Trocknung der ersten Komponente (2).

Auch der Einsatz von mehr als zwei Komponenten ist denkbar. Hierbei können beispielsweise die Nadelstümpfe (72) aus der weiteren Komponente (3) bestehen. Der Trägerstreifen (71) ist dann beispielsweise ein mit den Nadelstümpfen (72) gefügtes Band.

Nach dem Verfestigen der weiteren Komponente (3) kann das Mikronadelarray (61) aus der Form (21) entnommen werden. Die Figur 11 zeigt das Mikronadelarray (61) nach dem Entformen. Es besteht aus dem Trägerstreifen (71) und einer Vielzahl von starren Nadeln (73), die beispielsweise alle in die gleiche Richtung zeigen. Jede Nadel (73) umfasst einen an den Trägerstreifen (71) in einem Nadelanschlussquerschnitt (75) angeformten Nadelstumpf (72), an dessen dem Trägerstreifen (71) abgewandten Seite eine Spitze (62) gefügt ist. Im Ausführungsbeispiel stehen alle Nadeln (73) senkrecht zum Trägerstreifen (71). Der Trägerstreifen (71) kann elastisch verformbar ausgebildet sein. Sämtliche Nadeln (73) haben z.B. identische geometrische Abmessungen. Die dem Trägerstreifen (71) abgewandte Stirnseite einer jeden Nadel (73) wird durch eine kleinste Stirnfläche (63) einer Spitze (62) gebildet. Diese kleinste Stirnfläche (63) liegt in diesem Ausführungsbeispiel normal zu einer Mittellinie (74) der einzelnen Nadel (73).

Der Trägerstreifen (71) ist im Ausführungsbeispiel als Scheibe mit kreisförmigem Querschnitt ausgebildet. Er ragt über die Hüllkontur der Nadeln (73) im Nadelanschlussquerschnitt (75) hinaus. Der Trägerstreifen (71) kann, unabhängig von der Gestalt der genannten Hüllkontur der Nadeln (73), in einer Ebene normal zur Längsrichtung der Nadeln (73) einen kreisförmigen, elliptischen, rechteckigen, dreieckigen, etc. Querschnitt aufweisen.

Die einzelnen Nadeln (73) aus den erstarrten Füllungen (8) können auch zunächst ohne Verbindung durch den Trägerstreifen (71) hergestellt sein. Zur Entnahme der Nadeln (73) aus den Aufnahmen (25) wird dann beispielsweise ein Trägerstreifen (71) mit den Nadeln (73) verbunden. Beispielsweise wird der Trägerstreifen (71) hierbei adhäsiv mit den Nadeln (73) am Nadelanschlussquerschnitt (75) gefügt. Der Trägerstreifen (71) kann als Trägerplatte, als Trägerschicht, z.B. als polymere Klebefolie, als Klebeband, etc. ausgebildet sein. Das aus der Vorrichtung (10) entnommene Mikronadelarray (61) besteht auch in diesem Fall aus mindestens zwei Nadeln (73) und dem Trägerstreifen (71).

Nach der Entnahme des Mikronadelarrays (61) aus der Vorrichtung (10) kann diese z.B. erneut verwendet werden. Beispielsweise wird nach einer Reinigung der Vorrichtung (10) ein weiteres Mikronadelarray (61) mittels dieser Vorrichtung (10) hergestellt. Diese wiederholte Herstellung erfolgt, wie oben beschrieben.

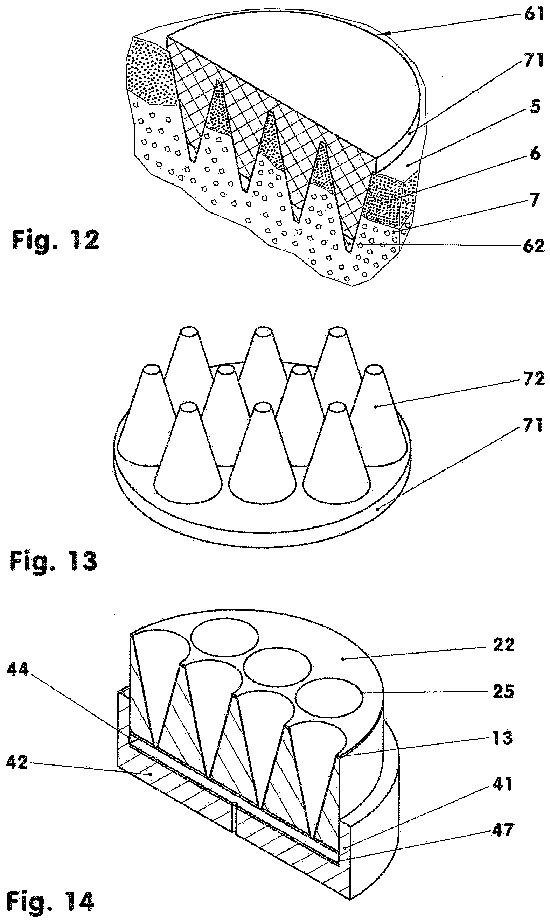

In der Figur 12 ist der Einsatz des Mikronadelarrays (61) dargestellt. Das Mikronadelarray (61) liegt mit dem Trägerstreifen (71) auf der Haut (5) eines Patienten auf. Der Trägerstreifen (71) mit den z.B. wirkstofffreien Nadelstümpfen (72) gewährleistet die geometrische Ausrichtung und Fixierung der Mikronadeln (61). Beispielsweise kann das Mikronadelarray (61) zusätzlich mittels eines Klebebands fixiert sein. Das Mikronadelarray (61) kann hierbei als Pflaster ausgebildet sein. Die starren Nadeln (73) durchdringen die Hornhaut (6) und ragen in mindestens eine weitere Hautschicht (7). Sie werden hierbei z.B. nicht plastisch verformt und brechen nicht. Nach dem Eindringen liegen die Spitzen (62) z.B. vollständig in der unter der Hornhaut (6) liegenden Hautschicht (7). Der Wirkstoff liegt beispielsweise quantitativ und räumlich definiert in den Spitzen (62) vor. Aufgrund der Umgebungsbedingungen in der Hautschicht (7) wird der Wirkstoff in die Hautschicht (7) abgegeben. Hierbei können sich die Spitzen (62) zersetzen. Beispielsweise lösen sie sich durch Aufnahme von Flüssigkeit in der Hautschicht (7) auf. An den Körper des Patienten wird somit der Wirkstoff abgegeben. Abhängig von der nach Applikation vorgesehenen Tragedauer, z.B. nach zehn Minuten, kann das Mikronadelarray (61) wieder von der Haut (5) des Patienten abgenommen werden.

Die Figur 13 zeigt das Mikronadelarray (61) aus der Figur 12 nach der Abnahme von der Haut (5) des Patienten. Das Mikronadelarray (61) besteht jetzt nur noch aus der verfestigten, unlöslichen weiteren Komponente (3) bzw. dem Trägerstreifen (71) und den daran hängenden Nadelstümpfen (72). Diese gebrauchten Mikronadelarrays (61) sind wegen der fehlenden Spitzen (62) gesichert gegen eine versehentliche Wiederverwendung. Sie können entsorgt werden. Hiermit wird u.a. die Sicherheit für den Patienten gewährleistet.

Es ist auch denkbar, sowohl die Nadelspitzen (62), als auch die Nadelbasis oder Nadelfüße und den Trägerstreifen (71), aus löslichen Komponenten oder Stoffgemischen herzustellen.

Alternativ ist es denkbar, die Nadelspitzen (62), die Nadelbasis und den Trägerstreifen (71) aus unlöslichen Komponenten oder Stoffgemischen herzustellen. Beispielsweise werden eine oder mehrere, zur therapeutisch und/oder diagnostischen Anwendung enthaltene Substanzen beispielsweise nach Quellung unter Feuchtigkeitsaufnahme an die umgebende Hautschicht abgegeben.

Die Nadelspitzen (62) können auch aus einer weniger schnell löslichen, die Nadelbasis und Trägerplatte (71) hingegen aus einer schnelllöslichen Komponente bestehen. Dies führt zu einer veränderten Freigabegeschwindigkeit von Wirkstoffen aus der jeweiligen Komponente.

Bei einer Vorrichtung (10) mit einer seitlichen Wirkstoffbefüllung kann beispielsweise die in der Figur 3 dargestellte Unterschale (41) eingesetzt werden. Die erste Komponente (2) ist dann in einem seitlich an die Unterschale (41) angeflanschten Behälter gelagert. Beispielsweise herrscht im Innenraum des Behälters Umgebungsdruck. Beim Öffnen eines Behälterventils fließt die im Behälter gelagerte flüssige erste Komponente (2) in die Unterschale (41). Von dort steigt sie durch die, die Zuführöffnungen (31) bildenden Spitzenflächen (28) in die Aufnahmen (25).

Der Anstieg erfolgt so lange, bis das Niveau in den Aufnahmen (25) mindestens so hoch ist wie im Behälter. Gegebenenfalls kann aufgrund von Kapillarwirkungen das Niveau der Flüssigkeit in den Aufnahmen (25) über das Niveau der zweiten Komponente (2) im Behälter ansteigen.

Die Figur 14 zeigt eine Vorrichtung (10) mit bereichsweise modifizierten Oberflächen. In der Form (21) sind die Innenflächen der Aufnahmen (25), die Oberseite (22) und die Unterseite (23) derart ausgebildet, dass diese Oberflächen in Bezug auf den Werkstoff der Spitzen (62) und der weiteren Komponente (3) dehäsiv sind. Die Haftreibung der genannten Werkstoffe gegenüber der Form (21) ist gegenüber einer nicht modifizierten Oberfläche vermindert. In der Unterschale (41) sind beispielsweise die der Form (21) zugewandte Innenseite (47) des Bodens (42) und die Innenfläche (44) des Randes (43) derart ausgebildet, dass das Anhaften des Ausgangsmaterials der Spitzen (62) verhindert wird.

Das Vermindern des Anhaftens kann mittels einer Beschichtung (13) der genannten Oberflächen, mittels des Auftrags eines Trennmittels und/oder mittels einer mechanischen Bearbeitung der Oberflächen erfolgen. Hierbei können die eingesetzten Verfahren und Beschichtungswerkstoffe und/oder Trennmittel unterschiedlich sein. Die Oberflächen, die nur mit der weiteren Komponente (3) in Kontakt kommen, können anders behandelt sein als die Oberflächen, die während der Herstellung des Mikronadelarrays (61) nur mit dem Werkstoff der Spitzen (62) in Kontakt kommen.

Bei einer Beschichtung (13) der Oberflächen kann diese beispielsweise aus Polytetraflourethylen, Polyethylen, Polypropylen, etc. bestehen. Als Trennmittel, das auf den genannten Oberflächen aufgetragen wird, kann z.B. ein Polysorbat oder ein anderes beispielsweise ölhaltiges Trennmittel eingesetzt werden. Bei einer mechanischen Behandlung der Oberflächen kann die Oberflächenrauheit beispielsweise mittels Elektropolieren, Coronabehandlung, Laserpolieren, etc. vermindert werden. Durch alle diese Maßnahmen werden Verluste bei der Dosierung des Mikronadelarrays (61) vermindert und eine beschädigungsfreie Entnahme des Mikronadelarrays (61) aus der Form (21) erleichtert.

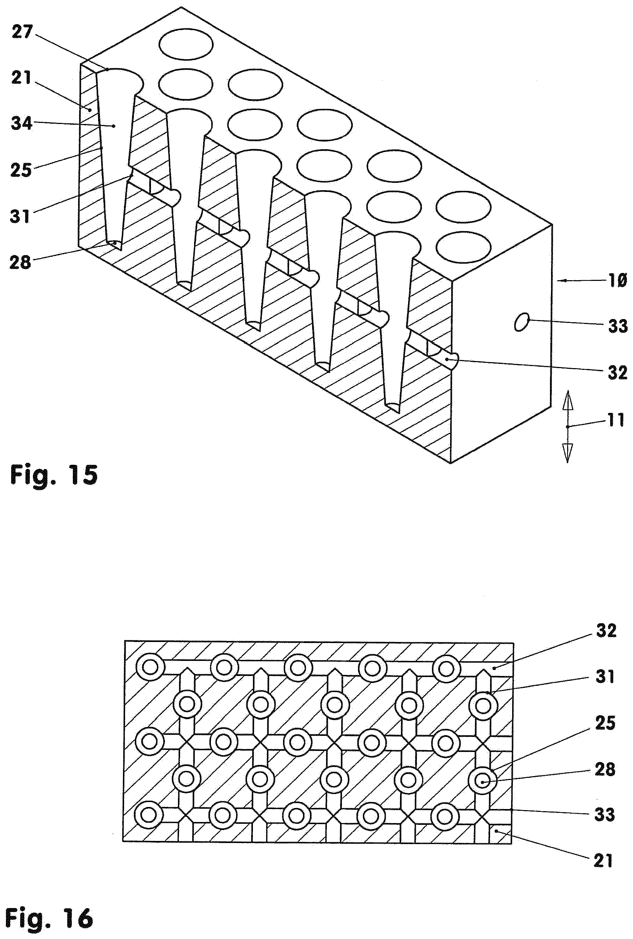

Die Figur 15 zeigt eine isometrische Schnittdarstellung einer Vorrichtung (10) ohne Unterschale (41). Die Vorrichtung (10) besteht aus der z.B. quaderförmigen Form (21). Die Aufnahmen (25) sind in mehreren Reihen versetzt zueinander angeordnet. Die Anordnung und die Gestalt der Aufnahmen (25) entspricht beispielsweise weitgehend der Anordnung und der Gestalt der im Zusammenhang mit den vorgenannten Ausführungsbeispielen beschriebenen Aufnahmen (25). An den jeweiligen Spitzenflächen (28) sind die Aufnahmen (25) verschlossen.

In den Innenmantelflächen (34) der Aufnahmen (25) münden Zuführöffnungen (31). Die Zuführöffnungen (31) liegen unterhalb der Basisflächen (27). Beispielsweise sind sie, ausgehend von den Spitzenflächen (28), in der unteren Hälfte der Höhe der Aufnahmen (25) in der Höhenrichtung (11) angeordnet. Auch eine Anordnung im unteren Drittel oder im unteren Fünftel der Höhe ist denkbar. Der Querschnitt der einzelnen Zuführöffnung (31) entspricht beispielsweise der Fläche der einzelnen Spitzenfläche (28).

Die Zuführöffnungen (31) sind mittels Zuführkanälen (32) mit von der Außenseite der Form (21) zugänglichen Zuführanschlüssen (33) verbunden. An den Zuführanschlüssen (33) ist beispielsweise ein hier nicht dargestelltes Reservoir angeschlossen. Dieses ist z.B. so ausgebildet, wie im Zusammenhang mit den vorher beschriebenen Ausführungsbeispielen erläutert. Beispielsweise bildet das Netz der sich z.B. kreuzenden Zuführkanäle (32) einen Zuführraum (51). Es ist auch denkbar, alle Zuführanschlüsse (33) an einer Außenseite der Form (21) anzuordnen.

Die Figur 16 zeigt eine Schnittdarstellung einer derartigen Form (21) mit innenliegenden Zuführkanälen (32). Die Schnittebene ist waagerecht, z.B. liegt sie parallel zur Ebene der Unterseite (23). Die Zufuhranschlüsse (33) liegen z.B. an zwei Seitenflächen der Form (21). Die Zuführkanäle (32) verbinden die einzelnen Aufnahmen (25) miteinander.

Nach dem Anschluss der Form (21) an das Reservoir fließt die erste Komponente (2) durch die Zuführkanäle (32) in die Aufnahmen (25). Aufgrund der miteinander kommunizierenden Aufnahmen (25) werden alle Aufnahmen (25) auf dasselbe Niveau befüllt. Beispielsweise liegt das sich bei einer drucklosen Befüllung mit der ersten Komponente (2) einstellende Füllniveau der Aufnahmen (25) innerhalb der Querschnittsfläche der Zuführöffnungen (31) und der mit diesen fluchtenden Zuführkanälen (32). Die erste Komponente (2) kann separat verfestigt werden. Hierbei wird beispielsweise der Anschluss der ersten Komponente (2) an der Zuführöffnung (31) abgetrennt. Der restliche Flüssigkeitsbestand aus dem Zuführraum (51) läuft entlang der Zuführkanäle (32) in die Aufnahmen (25). Beispielsweise werden die Zuführkanäle (32) geleert.

Das Befüllen der Aufnahmen (25) mit der weiteren Komponente (3) erfolgt, wie oben beschrieben. Beispielsweise anschließend werden die weitere Komponente (3) und der Trägerstreifen (71) verfestigt. Auch in diesem Ausführungsbeispiel können die Füllungen (8) gemeinsam verfestigt werden. Auch ist es denkbar, den Trägerstreifen (71) nach dem Verfestigen der Füllungen (8) auf die Nadeln (73) aufzukleben bzw. mit den Nadeln (73) formschlüssig oder stoffschlüssig zu verbinden.

Die weitere Komponente (3) kann auch in diesem Ausführungsbeispiel durch Füllöffnungen der Innenmantelflächen (34) der Aufnahmen (25) zugeführt werden, die in der Höhenrichtung (11) zwischen den Zuführöffnungen (31) und den Basisflächen (27) liegen.

Das Entnehmen der z.B. mehreren gemeinsam hergestellten Mikronadelarrays (61) aus der Form (21) erfolgt, wie oben beschrieben. Hierbei können gegebenenfalls restliche Verbindungen zwischen den Füllungen (8) und den Zuführkanälen (32) getrennt werden. Die Form (21) kann als wiederverwendbare oder als einmal verwendbare Form (21), z.B. als verlorene Form (21), eingesetzt werden.

Die Applikation des einzelnen Mikronadelarrays (61) erfolgt, wie oben beschrieben.

Die genannten Ausführungsbeispiele können auch miteinander kombiniert werden.

### Bezugszeichenliste:

- 1: Umgebung
- 2: erste Komponente, Lösung
- 3: weitere Komponente, Füllmasse, zweite Komponente
- 4: Walze, Verdichtungsvorrichtung
- 5: Haut
- 6: Hornhaut
- 7: weitere Hautschicht
- 8: Füllungen

- 10: Vorrichtung
- 11: Höhenrichtung
- 12: Verfahrrichtung
- 13: Beschichtung

- 21: Form, Gussform
- 22: Oberseite
- 23: Unterseite
- 24: Mantelfläche
- 25: Aufnahmen, Durchbrüche
- 26: Mittellinien
- 27: Basisflächen
- 28: Spitzenflächen
- 29: Mittelachse

- 31: Zuführöffnungen
- 32: Zuführkanäle
- 33: Zuführanschlüsse
- 34: Innenmantelflächen

- 41: Unterschale
- 42: Boden
- 43: Rand
- 44: Innenfläche
- 45: Außenfläche
- 46: Befüllanschluss
- 47: Innenseite
- 48: Kanäle, Bodenkanäle

- 51: Zuführraum
- 52: Ringspalt

- 61: Mikronadelarray
- 62: Spitzen
- 63: kleinste Stirnfläche
- 64: größte Stirnfläche
- 65: Mantelfläche von (62)

- 71: Trägerstreifen, Trägerplatte
- 72: Nadelstümpfe
- 73: Nadeln
- 74: Mittellinie von (73)
- 75: Nadelanschlussquerschnitt

## Patentansprüche

1. Verfahren zum Herstellen von Mikronadelarrays (61) in einer Form (21), die eine Vielzahl sich von einer obenliegenden Basisfläche (27) zu einer untenliegenden Spitzenfläche (28) verjüngender Aufnahmen (25) aufweist,
wobei
- mindestens zwei Aufnahmen (25) durch eine von der Basisfläche (27) beabstandete Zuführöffnung (31) hindurch eine erste Komponente (2) zugeführt wird,
- diese Aufnahmen (25) von oberhalb der Zuführöffnung (31) aus mit einer weiteren Komponente (3) befüllt werden,
- die zumindest aus der ersten Komponente (2) und aus der weiteren Komponente (3) gebildeten Füllungen (8) mindestens dieser zwei Aufnahmen (25) oberhalb der Basisflächen (27) miteinander verbunden werden, und
- nach dem Verfestigen der ersten Komponente (2) und der weiteren Komponente (3) das Mikronadelarray (61) mit den zu Nadeln (73) erstarrten Füllungen (8) aus der Form (21) entnommen wird ,
**dadurch gekennzeichnet, dass** die erste Komponente (2) durch die Spitzenflächen (28) zugeführt wird.

2. Verfahren nach Anspruch 1, **dadurch gekennzeichnet, dass** zumindest eine Vielzahl von Spitzenflächen (28) nach dem Zuführen der ersten Komponente (2) mittels einer relativ zur Form (21) beweglichen Unterschale (41) verschlossen werden.

3. Vorrichtung (10) zum Herstellen von Mikronadelarrays (61) mittels eines Verfahrens nach Anspruch 1 mit einer Form (21),
wobei die Form (21) eine Vielzahl von sich von einer obenliegenden Basisfläche (27) zu einer untenliegenden Spitzenfläche (28) verjüngender Aufnahmen (25) aufweist,
und wobei jede Aufnahme (25) eine von der Basisfläche (27) beabstandete Zuführöffnung (31) zum hindurch Zuführen einer ersten Komponente (2) hat, **dadurch gekennzeichnet, dass** die Zuführöffnung (31) der einzelnen Aufnahme (25) die Spitzenfläche (28) dieser Aufnahme (25) umfasst.

4. Vorrichtung (10) nach Anspruch 3, **dadurch gekennzeichnet, dass** die Zuführöffnung (31) die Aufnahme (25) mit einem ein Reservoir bildenden Zuführraum (51) verbindet.

5. Vorrichtung (10) nach Anspruch 3, **dadurch gekennzeichnet, dass** der Flächeninhalt der Querschnittsfläche der einzelnen Zuführöffnung (31) kleiner oder gleich 0,01 Quadratmillimeter beträgt.

6. Vorrichtung nach Anspruch 3, **dadurch gekennzeichnet, dass** die Spitzenflächen (28) in einer Ebene liegen.

7. Vorrichtung (10) nach Anspruch 3, **dadurch gekennzeichnet, dass** die Spitzenflächen (28) verschließbar sind.

8. Vorrichtung (10) nach Anspruch 3, **dadurch gekennzeichnet, dass** sie eine oberhalb der Form (21) angeordnete Verdichtungsvorrichtung (4) aufweist.

9. Vorrichtung (10) nach Anspruch 3, **dadurch gekennzeichnet, dass** die Flächen der Oberseite (22), der Unterseite (23) und der Aufnahmen (25) der Form (21) zumindest bereichsweise dehäsiv ausgebildet sind.

## Claims

1. A method of manufacturing microneedle arrays (61) in a mold (21), comprising a plurality of receptacles (25) tapering from an upper base surface (27) to a lower tip surface (28), wherein
- a first component (2) is fed to at least two receptacles (25) through a feed opening (31) spaced apart from the base surface (27),
- said receptacles (25) are filled with a further component (3) from above the feed opening (31),
- the fillings (8) formed at least from the first component (2) and from the further component (3) of at least these two receptacles (25) are connected to one another above the base surfaces (27), and
- after the first component (2) and the further component (3) have solidified, the microneedle array (61) comprising the fillings (8) solidified into needles (73) is removed from the mold (21),
**characterized in that** the first component (2) is fed through the tip surfaces (28).

2. The method according to claim 1, **characterized in that**, after feeding the first component (2), at least a plurality of tip surfaces (28) are closed by a subshell (41) movable relative to the mold (21).

3. A device (10) for manufacturing microneedle arrays (61) by means of a method according to claim 1, comprising a mold (21), wherein the mold (21) has a plurality of receptacles (25) tapering from an upper base surface (27) to a lower tip surface (28), and wherein each receptacle (25) has a feed opening (31) spaced apart from the base surface (27) for feeding a first component (2) therethrough, **characterized in that** the feed opening (31) of the individual receptacle (25) comprises the tip surface (28) of said receptacle (25).

4. The device (10) according to claim 3, **characterized in that** the feed opening (31) connects the receptacle (25) to a feed chamber (51) forming a reservoir.

5. The device (10) according to claim 3, **characterized in that** the surface area of the cross-sectional surface of the individual feed opening (31) is smaller than or equal to 0.01 square millimeters.

6. The device according to claim 3, **characterized in that** the tip surfaces (28) are located in one plane.

7. The device (10) according to claim 3, **characterized in that** the tip surfaces (28) are closable.

8. The device (10) according to claim 3, **characterized in that** the device comprises a compression device (4) arranged above the mold (21).

9. The device (10) according to claim 3, **characterized in that** the surfaces of the upper side (22), the lower side (23) and the receptacles (25) of the mold (21) are designed to be non-adhesive in at least some areas.

## Revendications

1. Procédé de production de matrices de micro-aiguilles (61) dans un moule (21) présentant une pluralité de réceptacles (25) se rétrécissant depuis une surface de base (27) supérieure jusqu'à une surface de pointe (28) inférieure, dans lequel
au moins deux réceptacles (25) sont alimentés en un premier composant (2) à travers un orifice d'alimentation (31) distant de la surface de base (27),
lesdits réceptacles (25) sont remplis par le haut de l'orifice d'alimentation (31) avec un composant supplémentaire (3),
les remplissages (8), formés au moins du premier composant (2) et du composant (3) supplémentaire, d'au moins lesdits deux réceptacles (25) sont reliés l'un à l'autre au-dessus des surfaces de base (27), et
après la solidification du premier composant (2) et du composant supplémentaire (3), la matrice de micro-aiguilles (61) est retirée du moule (21) avec les remplissages (8) solidifiés en aiguilles (73), **caractérisé en ce que**
le premier composant (2) est amené à travers les surfaces de pointe (28).

2. Procédé selon la revendication 1, **caractérisé en ce qu'**au moins une pluralité de surfaces de pointe (28) sont fermées après l'apport du premier composant (2), au moyen d'une coque inférieure (41) mobile par rapport au moule (21).

3. Dispositif (10) permettant de produire des matrices de micro-aiguilles (61) au moyen d'un procédé selon la revendication 1, comprenant un moule (21), dans lequel
le moule (21) présente une pluralité de réceptacles (25) se rétrécissant depuis une surface de base (27) supérieure jusqu'à une surface de pointe (28) inférieure,
et dans lequel chaque réceptacle (25) possède un orifice d'alimentation (31) distant de la surface de base (27) et permettant d'amener un premier composant (2) à travers celui-ci, **caractérisé en ce que**
l'orifice d'alimentation (31) du réceptacle (25) individuel englobe la surface de pointe (28) dudit réceptacle (25).

4. Dispositif (10) selon la revendication 3, **caractérisé en ce que** l'orifice d'alimentation (31) relie le réceptacle (25) à une chambre d'alimentation (51) formant un réservoir.

5. Dispositif (10) selon la revendication 3, **caractérisé en ce que** la superficie de la section transversale de l'orifice d'alimentation (31) individuel est inférieure ou égale à 0,01 millimètre carré.

6. Dispositif selon la revendication 3, **caractérisé en ce que** les surfaces de pointe (28) se situent dans un plan.

7. Dispositif (10) selon la revendication 3, **caractérisé en ce que** les surfaces de pointe (28) peuvent être fermées.

8. Dispositif (10) selon la revendication 3, **caractérisé en ce qu'**il présente un dispositif de compression (4) agencé au-dessus du moule (21).

9. Dispositif (10) selon la revendication 3, **caractérisé en ce que** les surfaces de la face supérieure (22), de la face inférieure (23) et des réceptacles (25) du moule (21) sont conçues pour être au moins partiellement antiadhésives.
